# EUROPEAN PATENT APPLICATION

(11) **EP 4 338 746 A1**
(43) Date of publication of application: **20.03.2024**
(21) Application number: 22807904.2
(22) Date of filing: 13.05.2022
(51) Int. Cl.: A61K 36/315, A61P 1/00, A61P 29/00

(54) **COMPOSITION CONTAINING ISATIS TINCTORIA LEAF EXTRACT FOR PREVENTING, RELIEVING, OR TREATING INFLAMMATORY BOWEL DISEASE**

(30) Priority: 14.05.2021 KR 20210062846
(71) Applicant: Mthera Pharma Co., Ltd., Seoul 07793 (KR)
(72) Inventor: SOHN, Mi Won, Yongin-si Gyeonggi-do 16822 (KR); KIM, Sinyeon, Seoul 07063 (KR); CHOI, Jin Gyu, Bucheon-si Gyeonggi-do 14694 (KR); KIM, Se Woong, Seoul 07522 (KR); PARK, Sang Cheol, Seoul 03115 (KR); LEE, Mijeong, Seoul 07792 (KR)
(74) Representative: Duncombe, Jessica
(86) International application number: PCT/KR2022/006932
(87) International publication number: WO 2022/240257

(57) **Abstract**

The present invention relates to a pharmaceutical composition for preventing or treating inflammatory bowel disease, comprising an *Isatidis Folium* extract. In addition, the present invention relates to a food composition or animal feed composition for preventing or improving inflammatory bowel disease, comprising the *Isatidis Folium* extract.

## Description

### Technical Field

The present invention relates to a pharmaceutical composition for preventing or treating inflammatory bowel disease, comprising an *Isatidis Folium* extract. In addition, the present invention relates to a food composition or animal feed composition for preventing or improving inflammatory bowel disease, comprising the *Isatidis Folium* extract.

### Background Art

Inflammatory bowel disease (IBD) is a disease that causes chronic inflammation of an unknown cause in the intestinal tract, and shows a clinical course that is progressed with repeated exacerbation and improvement. Ulcerative colitis and Crohn's disease are representative diseases. Although the cause or pathophysiology of inflammatory bowel disease is not yet clearly known, it is assumed that genetic factors, environmental factors such as intestinal bacteria or food, immunological factors, and the like are involved in the development mechanism in a complex manner. Sustained or inappropriate activation of the intestinal immune system plays an important role in the pathophysiology of chronic mucosal inflammation, particularly by infiltration of neutrophils, macrophages, lymphocytes and mast cells, eventually leading to mucosal destruction and ulceration. Infiltrated and activated neutrophils are an important cause of reactive oxygen species and reactive nitrogen species, and these reactive species are cytotoxic substances that induce oxidative stress that degrades cross-linked proteins, lipids, and nucleic acids, resulting in epithelial dysfunction and damage.

Since a fundamental treatment for inflammatory bowel disease has not yet been established, drugs capable of alleviating symptoms are being used. Mainly, 5-aminosalicylic acid (5-ASA) type drugs that block the production of prostaglandins, such as sulfasalazine, or steroid type immunosuppressants are used.

Sulfasalazine is prone to cause side effects or adverse effects such as abdominal fullness, headache, rash, liver disease, leukopenia, agranulocytosis, and male infertility. In addition, it is unclear whether sulfasalazine has a sufficient recurrence inhibitory effect in patients who have undergone an incision in the affected part of the intestine or in patients who are in remission.

Steroid type immunosuppressants are adrenocortical steroids, which have been recognized for their short-term effects, but cannot enhance long-term prognosis. In addition, in terms of side effects such as induced infectious diseases, secondary adrenocortical insufficiency, peptic ulcer, diabetes, mental disorder, and steroidal nephropathy, there is a limitation that it should be used only in acute cases.

There is no reliable treatment for inflammatory bowel disease yet, so there is a need to develop new therapeutic agents that are inexpensive and have excellent therapeutic effects with fewer side effects. As an example, a composition for preventing or treating inflammatory bowel disease using natural product extracts (Korean Patent Registration No. 10-1710730) are being developed.

On the other hand, *Isatidis Folium* is a leaf of *Isatis indigotica* Fortune, a biennial herb belonging to the family Cruciferae, and is collected in summer and autumn. The whole plant is called *Isatis indigotica* Fortune and the root is called *Isatidis Radix*, and it is known to be effective in influenza, hepatitis, acute pneumonia, jaundice, and the like.

Accordingly, the present inventors have made efforts to develop a novel therapeutic agent having excellent effects on inflammatory bowel disease using natural products, and as a result, have found that an *Isatidis Folium* extract can improve inflammatory bowel disease. Based on the above, the present inventors completed the present invention.

### Detailed Description of Invention

### Technical Problem

An object of the present invention is to provide a pharmaceutical composition for preventing or treating inflammatory bowel disease, comprising an *Isatidis Folium* extract.

In addition, another object of the present invention is to provide a food composition for preventing or improving inflammatory bowel disease, comprising an *Isatidis Folium* extract.

In addition, another object of the present invention is to provide an animal feed composition for preventing or improving inflammatory bowel disease, comprising an *Isatidis Folium* extract.

### Solution to Problem

The present invention provides a pharmaceutical composition for preventing or treating inflammatory bowel disease, comprising an *Isatidis Folium* extract.

In addition, the present invention provides a food composition for preventing or improving inflammatory bowel disease, comprising an *Isatidis Folium* extract.

In addition, the present invention provides an animal feed composition for preventing or improving inflammatory bowel disease, comprising an *Isatidis Folium* extract.

The extract may be extracted with a solvent selected from the group consisting of water, C1 to C6 alcohol, and mixed solvents thereof, and it may be preferably a 0.01% to 90% ethanol extract, more preferably a 60% to 80% ethanol extract, most preferably a 70% ethanol extract.

The composition can inhibit a decrease in body weight or the length of the large intestine, and can inhibit an increase in the weight or length of the spleen.

### Effects of Invention

The composition comprising an *Isatidis Folium* extract of the present invention exhibits an excellent therapeutic effect on inflammatory bowel disease and can therefore be used as a novel therapeutic agent for inflammatory bowel disease.

In addition, the composition of the present invention is an extract of a natural product and has few side effects and is safe, so it can be used very usefully for patients with inflammatory bowel disease caused by various causes.

### Brief Description of Drawings

Figure 1 shows the rate of change in body weight in mice according to the administration of the extract of the present invention.
Figure 2 shows the length of the large intestine in mice according to the administration of the extract of the present invention.
Figure 3 shows the spleen ratio measured by the weight and length of the spleen in each group according to the administration of the extract of the present invention compared to that of the negative control group of mice.
Figure 4 shows the disease activity index (DAI) of mice according to the administration of the extract of the present invention.

### Best Mode for Carrying out the Invention

Hereinafter, with reference to the accompanying drawings, embodiments and examples of the present invention will be described in detail so that those of ordinary skill in the art to which the present invention belongs can easily carry out. However, the present invention may be implemented in various forms and is not limited to the embodiments and examples described herein.

Throughout the present specification, when a certain part "includes" a certain component, it means that other components may be further included, rather than excluding other components, unless otherwise stated.

The present invention provides a composition for preventing, improving or treating inflammatory bowel disease, comprising an *Isatidis Folium* extract.

The composition may be a pharmaceutical composition, a food composition or an animal feed composition.

*Isatidis Folium* includes a dried leaf of *Isatis indigotica* Fortune.

*Isatidis Folium* of the present invention is a leaf of *Isatis indigotica* Fortune, a biennial herb belonging to the family Cruciferae, and is collected in summer and autumn. The whole plant is called *Isatis indigotica* Fortune and the root is called *Isatidis Radix.*

The extract used in the present invention can be obtained using a conventional extraction solvent known in the art. A polar solvent or a non-polar solvent may be used as the extraction solvent. The polar solvent includes water, C1 to C6 alcohol (for example, methanol, ethanol, propanol, butanol, n-propanol, iso-propanol and n-butanol, etc.), acetic acid, or a mixture of the above polar solvents. The non-polar solvent includes acetone, acetonitrile, ethyl acetate, methyl acetate, butyl acetate, fluoroalkane, hexane, ether, chloroform, dichloromethane or a mixture of the above non-polar solvents.

The extract of the present invention may be extracted with a solvent selected from the group consisting of water, C1 to C6 alcohol, and mixed solvents thereof, and it may be preferably a 0.01% to 90% ethanol extract, more preferably a 60% to 80% ethanol extract, most preferably a 70% ethanol extract.

The extract used in the present invention may be extracted through hot water extraction, cold extraction, reflux cooling extraction, ultrasonic extraction, or a conventional extraction method known in the art.

As used herein, the term "extract" means what is commonly used as a crude extract in the art, but also includes a fraction obtained by further fractionating the extract in a broad sense. That is, the extract includes not only those obtained using the above-described solvent, but also those obtained by additionally applying a purification process thereto. For example, the extract of the present invention also includes a fraction obtained by passing the extract through an ultrafiltration membrane having a certain molecular weight cut-off value, and a fraction obtained through various purification methods that are additional performed, such as separation by various chromatographies (designed for separation depending on size, charge, hydrophobicity or affinity).

The pharmaceutical composition of the present invention can be parenterally administered or orally administered depending on the desired method, and the range of dosage varies depending on the patient's body weight, age, sex, health condition, and diet, the administration time, the administration method, the excretion rate, and the severity of the disease and the like. In addition, the therapeutically effective amount of the composition may vary depending on the administration method, the target site, and the condition of the patient, and when used in the human body, the dosage should be determined in an appropriate amount in consideration of safety and efficiency.

The food composition of the present invention may be a health functional food, a dairy product, a fermented product, or a food additive.

The health functional food refers to a food manufactured and processed using raw materials or ingredients having useful functionality for the human body, and "functionality" refers to regulating nutrients for the structure and function of the human body or ingesting nutrients for the purpose of obtaining useful effects for health purposes such as physiological functions and the like.

The animal feed composition of the present invention can be ingested by all non-human animals, such as non-human primates, sheep, dogs, cattle, horses and the like.

As used herein, the term "prevention" refers to any action that inhibits or delays a disease and the like by administration of the composition according to the present invention, and "improvement" refers to any action that at least reduces a parameter related to a condition to be treated by administration of the composition of the present invention, for example, the severity of symptoms, and "treatment" refers to any action that ameliorates or beneficially changes the symptoms of the subject who is suspected to have or has developed a disease by administration of the composition of the present invention.

Hereinafter, the present invention will be described in more detail through the examples, but the following examples are for illustrative purposes only and are not intended to limit the scope of the present invention.

### [Preparation Example]

### Preparation of Isatidis Folium extract

To the washed and dried *Isatidis Folium* was added a 10-fold amount of a 70% ethanol aqueous solution and extracted at room temperature for 72 hours. After extraction, the extract was filtered under reduced pressure with a 5 µm filter paper and then concentrated under reduced pressure at 50 to 65 °C to obtain a herbal extract in powder form.

### [Example 1]

### Evaluation of body weight change in mice according to administration of extract

6-week-old C57BL/6N male mice were purchased and pre-bred for one week, and then they were randomly divided into a total of 4 groups of 8 mice per group, and experiments were conducted as shown in Table 1 below.

2.5% dextran sodium sulfate (DSS) diluted in sterile water was administered in drinking water for a total of 8 days, and 5-aminosalicylic acid (5-ASA), which is a therapeutic agent for inflammatory bowel disease, or the *Isatidis Folium* extract of the present invention was orally administered for a total of 10 days from 2 days prior to the day of administration with DSS.

**[Table 1]**

| | |
|---|---|
| Normal group (NC) | Oral administration of sterile water |
| Negative control group | administration of 2.5% DSS |
| Positive control group | administration of 2.5% DSS + oral administration of 5-aminosalicylic acid (5-ASA) at a concentration of 100 mg/kg |
| Experimental group | administration of 2.5% DSS + oral administration of *Isatidis Folium* extract at a concentration of 300 mg/kg |

Body weight loss is a representative symptom of inflammatory bowel disease (Scientific Reports volume 10, Article number: 3829 (2020)). The body weight of the mouse was measured once a day at the same time (10 am) using an electronic scale. The rate of change in body weight of each group was obtained by summing the body weight of 8 mice per group and then dividing by 8. The average body weight immediately before the start of administration of 2.5% DSS in drinking water was set as 100%, and the average body weight of each group was calculated every day to measure the rate of change in body weight. Significance was verified by performing a Mann-Whitney test for each group between the negative control group to which 2.5% DSS was administered in drinking water and the experimental group.

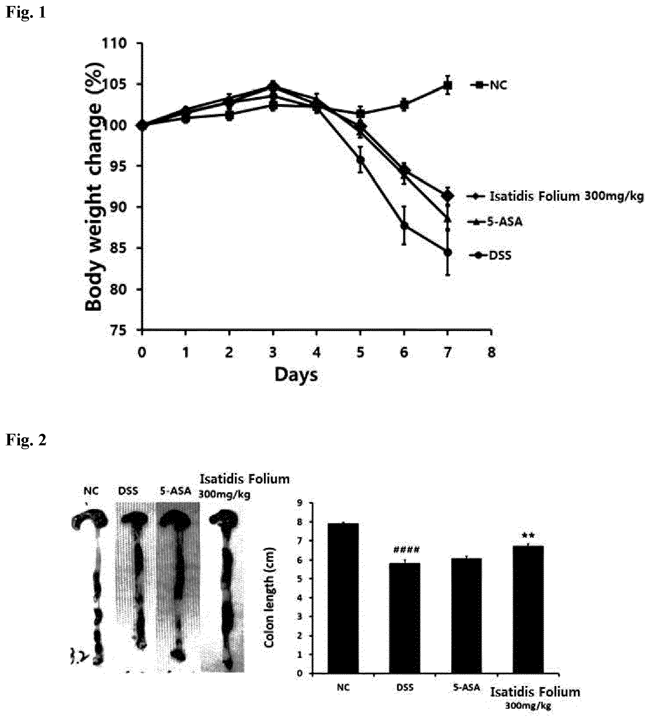

As shown in Figure 1, as a result of measuring the rate of change in body weight of mice according to the administration of the extract, the normal group showed 104.89±1.09 %, whereas the negative control group showed 84.50±2.78 %, indicating that the body weight was decreased compared to that of the normal group. In addition, the positive control group administered with 5-ASA showed 88.62±1.52 %, whereas the experimental group administered with an *Isatidis Folium* extract showed 91.37±1.02 %, indicating that a decrease in body weight was inhibited. It was confirmed that a decrease in body weight was more excellently inhibited compared to that of the positive control group.

Therefore, it can be seen that the *Isatidis Folium* extract of the present invention has an effect of inhibiting a decrease in body weight in mice.

### [Example 2]

### Evaluation of length of large intestine in mice according to administration of extract

It is known that there is a sufficient correlation between the shortened large intestine and histological changes, and the length of the large intestine is commonly used as a morphological parameter of the degree of inflammation. Therefore, in order to evaluate the length of the large intestine of the mouse according to the administration of the *Isatidis Folium* extract of the present invention, the mouse of Example 1 was sacrificed on the last day of each administration, and then the length of the large intestine was measured, and the average length is shown in Figure 2.

As shown in Figure 2, as a result of measuring the length of the large intestine in mice according to the administration of the extract, the length of the large intestine in the normal group was 7.89±0.10 cm, whereas the length of the large intestine in the negative control group was 5.81±0.21 cm, indicating that the length of the large intestine was decreased compared to that of the normal group. In addition, the length of the large intestine in the positive control group was 6.05±0.16 cm, whereas the length of the large intestine in the experimental group administered with an *Isatidis Folium* extract was 6.73±0.13 cm, indicating that a decrease in the length of the large intestine was inhibited. It was confirmed that a decrease in the length of the large intestine was more excellently inhibited compared to that of the positive control group.

Therefore, it can be seen that the *Isatidis Folium* extract of the present invention has an effect of inhibiting a decrease in the length of the large intestine in mice.

### [Example 3]

### Evaluation of level of inflammation in spleen of mice according to administration of extract

A representative symptom that can occur in relation to inflammatory bowel disease includes splenitis. As such, when the spleen becomes inflamed, the size of the spleen is increased by its nature (Nutrients 2019, 11(11), 2776), and in fact, it is known that the spleen becomes enlarged in the model of IBD induced after DSS treatment (Biol. Pharm. Bull. 43, 450-457 (2020)). Therefore, in order to evaluate the level of inflammation in the spleen of mice according to the administration of the *Isatidis Folium* extract of the present invention, the mouse of Example 1 was sacrificed on the last day of each administration, and then the weight and length of the spleen of each group compared to those of the negative control group were measured, and the spleen ratio is shown.

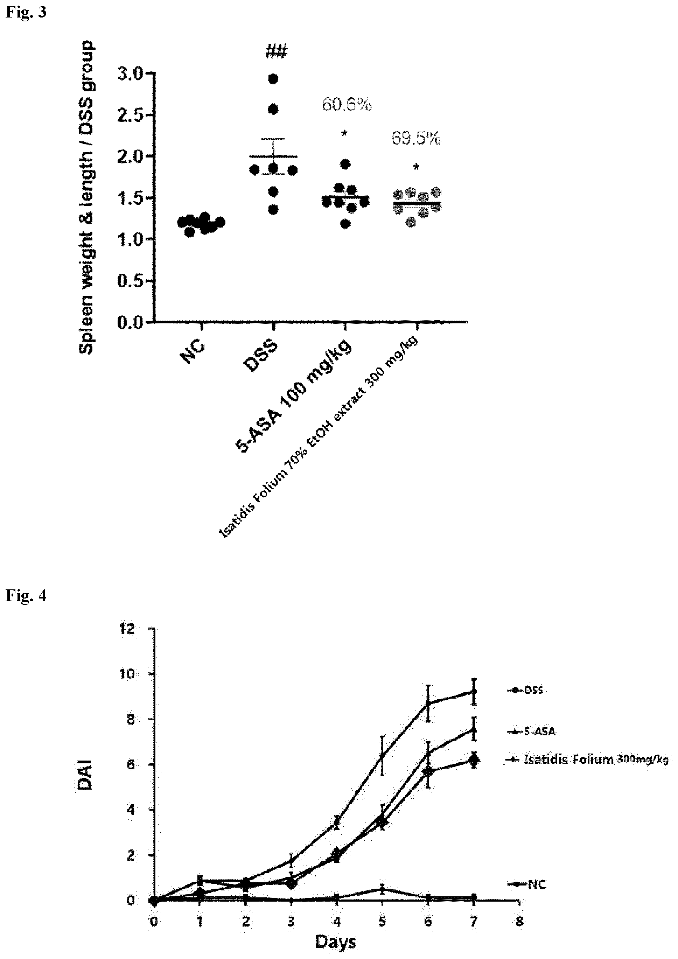

As shown in Figure 3, as a result of measuring the weight and length of the spleen in mice according to the administration of the extract, it was found that the weight and length of the spleen in negative control group were increased compared to those of the normal group, whereas the weight and length of the spleen in the positive control group and the experimental group were decreased compared to those of the negative control group. The weight and length of the spleen in the positive control group were decreased by 60.6 %, and the weight and length of the spleen in the experimental group administered with an *Isatidis Folium* extract were decreased by 69.5 % compared to those of the negative control group. It was confirmed that the weight and length of the spleen in the experimental group administered with an *Isatidis Folium* extract were more excellently decreased compared to those of the positive control group.

Therefore, it can be seen that the *Isatidis Folium* extract of the present invention has an effect of improving the symptoms of spleen enlargement due to spleen inflammation caused by inflammatory bowel disease in mice.

### [Example 4]

### Evaluation of disease activity index (DAI) in mice according to administration of extract

In order to measure the intensity of inflammatory bowel disease in the inflammatory bowel disease animal model treated with the administration method of Example 1 above, the disease activity index (DAI) in Table 2 below was measured by checking change in body weight, stool hardness, and the presence or absence of bloody stool visually observed in stool or anus every day during the administration period according to the disease activity index grade, and the results are shown in Figure 4.

**[Table 2]**

| Disease activity index score | | | | | |
|---|---|---|---|---|---|
| Body weight loss | Score | Shape of tool | Score | Bloody stool | Score |
| <1% | 0 | firm and hard | 0 | none | 0 |
| 1-5% | 1 | hard but sticky | 1 | hidden | 1 |
| 5-10% | 2 | soft but retained shape | 2 | visible | 2 |
| 10-15% | 3 | soft and lost shape | 3 | rectal bleeding | 3 |
| 15-20% | 4 | liquid and shapeless | 4 | - | - |

As shown in Figure 4, as a result of measuring the disease activity index in mice according to the administration of the extract, it was found that the disease activity index was significantly increased in the negative control group. On the other hand, it was found that the disease activity index in the experimental group administered with an *Isatidis Folium* extract was significantly decreased compared to that of the negative control group, indicating that it has a more excellent effect of reducing the disease activity index compared to that of the positive control group.

Therefore, it was confirmed that the *Isatidis Folium* extract of the present invention has an effect of improving the disease activity index of inflammatory bowel disease in an inflammatory bowel disease animal model.

## Claims

1. A pharmaceutical composition for preventing or treating inflammatory bowel disease, comprising an *Isatidis Folium* extract.

2. The pharmaceutical composition for preventing or treating inflammatory bowel disease according to claim 1, wherein the extract is extracted with a solvent selected from the group consisting of water, C1 to C6 alcohol, and mixed solvents thereof.

3. The pharmaceutical composition for preventing or treating inflammatory bowel disease according to claim 1, wherein the extract is a 0.01% to 90% ethanol extract.

4. The pharmaceutical composition for preventing or treating inflammatory bowel disease according to claim 1, wherein the extract is a 60% to 80% ethanol extract.

5. The pharmaceutical composition for preventing or treating inflammatory bowel disease according to claim 1, wherein the extract is a 70% ethanol extract.

6. The pharmaceutical composition for preventing or treating inflammatory bowel disease according to claim 1, wherein the composition inhibits a decrease in body weight or the length of the large intestine.

7. The pharmaceutical composition for preventing or treating inflammatory bowel disease according to claim 1, wherein the composition inhibits an increase in the weight or length of the spleen.

8. A food composition for preventing or improving inflammatory bowel disease, comprising an *Isatidis Folium* extract.

9. An animal feed composition for preventing or improving inflammatory bowel disease, comprising an *Isatidis Folium* extract.
